# EUROPEAN PATENT APPLICATION

(11) **EP 4 318 381 A1**
(43) Date of publication of application: **07.02.2024**
(21) Application number: 23189177.1
(22) Date of filing: 02.08.2023
(51) Int. Cl.: G06T 5/00

(54) **IMAGE CONVERSION METHOD AND DEVICE**

(30) Priority: 04.08.2022 KR 20220097571
(71) Applicant: Medicalip Co., Ltd., Gangwon-do 24341 (KR)
(72) Inventor: KIM, Jong Min, Yongin-si, Gyeonggi-do (KR); CHUNG, Han Jae, Seoul (KR); HAM, Seung Min, Seoul (KR); PARK, Sang Joon, Seoul (KR)
(74) Representative: Patentanwälte Ruff, Wilhelm, Beier, Dauster & Partner mbB

(57) **Abstract**

A method and a device for converting a non-contrast image into a contrast image are disclosed. The image conversion device converts the non-contrast image into the contrast image by using a deep learning network trained with learning data including one or more contrast learning images and one or more non-contrast learning images.

## Description

The invention relates to an image conversion method and device, more specificially to a method and a device for converting a medical image, and more particularly, to a method and a device for converting a non-contrast image into a contrast image by using a deep learning network.

This application is based on and claims priority under 35 U.S.C. §119 to Korean Patent Application No. 10-2022-0097571, filed on August 4, 2022, in the Korean Intellectual Property Office, the disclosure of which is incorporated by reference herein in its entirety.The invention was supported by the "Critical Care Patient Specialized Big Data Construction and Al-based CDSS Development" project hosted by Seoul National University Hospital (Task identification number: HI21C1074, Assignment number: HI21C1074050021).

In order to distinguish lesions more clearly during diagnosis or treatment, computed tomography (CT) or magnetic resonance imaging (MRI) is scanned by administrating a contrast medium. A medical image scanned by projecting a contrast medium has high tissue contrast, and thus, lesions may be clearly distinguished. However, a contrast medium is nephrotoxic. For example, a gadolinium contrast medium used in MRI is more nephrotoxic than an iodinated contrast medium used in CT scans, and thus, the contrast medium may not be used when a renal function is reduced. Therefore, elderly patients or patients with renal function problems have no choice but to take non-contrast images.

It is the technical problem underlying the invention to provide an image conversion method and device which are capable to avoid or at least alleviate difficulties of prior art image conversion methods and devices.

The invention solves this problem by providing an image conversion method having the features of claim 1 and an image conversion device having the features of claim 11. Advantageous embodiments of the invention are mentioned in the dependent claims, the wording of which is herewith incorporated into the description by reference to avoid unnecessary text repetitions. Additional aspects will be set forth in part in the description which follows and, in part, will be apparent from the description, or may be learned by practice of the presented embodiments of the invention.

Provided are an image conversion method and device capable of generating and outputting a contrast image from a non-contrast image by using a deep learning network.

According to the invention, the image conversion method includes inputting a non-contrast image to a deep learning network, and generating and outputting a contrast image through the deep learning network, wherein the deep learning network is trained with learning data including one or more contrast learning images and one or more non-contrast learning images.

According to the invention, the image conversion device includes an input unit configured to input a non-contrast image to a deep learning network, and a conversion unit configured to generate and output a contrast image through the deep learning network, wherein the deep learning network is trained with learning data including one or more contrast learning images and one or more non-contrast learning images.

The above and other aspects, features, and advantages of embodiments of the invention will be more apparent from the following description taken in conjunction with the accompanying drawings, in which:
FIG. 1 is a diagram showing an example of an image conversion device according to an embodiment;
FIG. 2 is a diagram showing an example of a deep learning network according to an embodiment;
FIG. 3 is a diagram showing an example of a method of generating a maximum intensity projection (MIP) image according to an embodiment;
FIG. 4 is a diagram showing another example of a method of generating a MIP image according to an embodiment;
FIG. 5 is a diagram showing a result of a deep learning network trained by using a MIP image according to an embodiment;
FIG. 6 is a diagram showing an example of implementing a deep learning network as a U-Net according to an embodiment;
FIG. 7 is a diagram showing an example of a method of training a deep learning network according to an embodiment;
FIG. 8 is a diagram showing another example of a method of training a deep learning network according to an embodiment;
FIG. 9 is a flowchart illustrating an example of a method of converting a non-contrast image into a contrast image according to an embodiment; and
FIG. 10 is a diagram showing a configuration of an example of an image conversion device according to an embodiment.

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. Hereinafter, an image conversion method and device according to an embodiment will be described in detail with reference to the accompanying drawings.

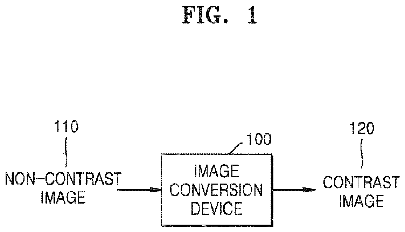

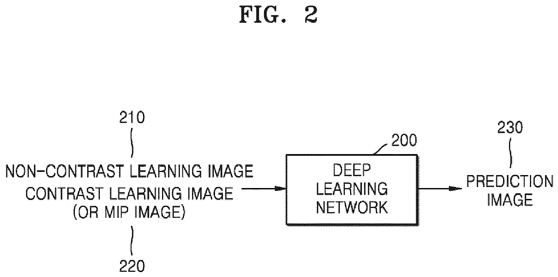

FIG. 1 is a diagram showing an example of an image conversion device according to an embodiment. Referring to FIG. 1, when a non-contrast image 110 is received, an image conversion device 100 converts the non-contrast image 110 into a contrast image 120 and outputs the contrast image 120 by using a deep learning network. Here, the non-contrast image 110 is an image captured during computed tomography (CT) or magnetic resonance imaging (MRI) without administering a contrast medium. The deep learning network used to convert the non-contrast image 110 into the contrast image 120 and output the contrast image 120 is described below in FIG. 2.

FIG. 2 is a diagram showing an example of a deep learning network according to an embodiment. Referring to FIG. 2, a deep learning network 200 may be implemented with various artificial neural networks, such as a convolutional neural network (CNN) and U-Net, according to the related art, and is not limited to a specific example. In addition, hereinafter, for convenience of description, a contrast image and a non-contrast image of learning data are named a contrast learning image 210 and a non-contrast learning image, respectively, and the contrast image output by the deep learning network 200 is named a prediction image 230.

The deep learning network 200 may be trained by using learning data including a dataset of the non-contrast learning images 210 and contrast learning images 220 that are a ground truth. The number of datasets included in the learning data may vary according to an embodiment. When the non-contrast learning image 210 is received, the deep learning network 200 outputs the prediction image 230, and performs a learning process of comparing the prediction image 230 with the contrast learning image 220 of the learning data, and adjusting an internal parameter so that an error between the prediction image 230 and the contrast learning image 220 is minimized.

The non-contrast learning image 210 and the contrast learning image 220 of the learning data may be images directly captured through CT or MRI or processed images for learning. For example, a non-contrast image and a contrast image directly captured from a plurality of patients may be used as the learning data in the embodiment. The contrast learning image 220 of the learning data may be a T1 image of MRI captured by administering a contrast medium.

Contrast images captured by administering the contrast medium may have different contrast enhancement degrees over time for each patient. For example, a contrast enhancement degree of a contrast image of a first patient and a contrast image of a second patient captured at the same time point after administration of the contrast medium may be different from each other. There is a disadvantage in that the learning performance of a deep learning network is reduced when such contrast images having different contrast enhancement degrees are used as learning data.

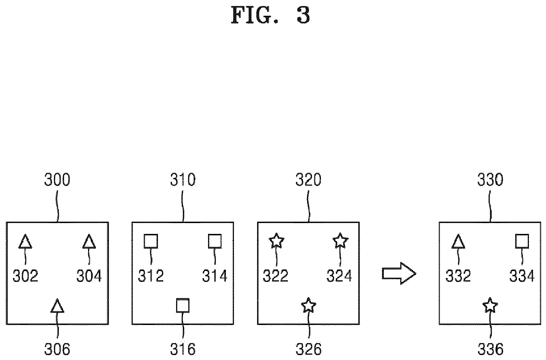

In order to solve this problem, the deep learning network 200 may use a maximum intensity projection (MIP) image 220 obtained by processing a plurality of contrast learning images as the learning data instead of using contrast learning images as they are. The MIP image 220 is an image including pixels having the highest brightness among pixels of the plurality of contrast learning images, which is described again with reference to FIG. 3. The deep learning network 200 may be trained by using the learning data including a dataset of the non-contrast learning image 210 and the MIP image 220. That is, the deep learning network 200 may be trained through a process of comparing the prediction image 230 with the MIP image 220 that is a ground truth of the learning data and minimizing an error between the prediction image 230 and the MIP image 220.

FIG. 3 is a diagram showing an example of a method of generating an MIP image according to an embodiment. Referring to FIG. 3, the image conversion device 100 may generate an MIP image 330 by selecting a pixel having the highest brightness among pixels of a plurality of contrast learning images. A three-dimension (3D) medical image may be expressed in units of voxels, and thus, a pixel in the embodiment may mean a voxel according to an embodiment.

In the embodiment, to help understanding, some pixels of first to third contrast learning images 300, 310, and 320 are shown in different shapes. For example, pixels 302, 304, and 306 of the first contrast learning image 300 are displayed as triangles, pixels 312, 314, and 316 of the second contrast learning image 310 are displayed as squares, and pixels 322, 324, and 326 of the third contrast learning image 320 are displayed as star shapes.

When a brightness value of the pixel 302 of the first contrast learning image 300 is the greatest among the pixels 302, 312, and 322 of the first to third contrast learning images 300, 310, and 320 existing at a first position, the image conversion device 100 selects a brightness value of the pixel 312 of the first contrast learning image 300 as a brightness value of a pixel 332 of the MIP image 330 at the first position. In the same way, when a brightness value of the pixel 314 of the second contrast learning image 310 is the greatest among the pixels 304, 314, and 324 of the first to third contrast learning images 300, 310, and 320 existing at a second position, the image conversion device 100 selects a brightness value of the pixel 314 of the second contrast learning image 310 as a brightness value of the pixel 332 of the MIP image 330 at the second position. In other words, when the plurality of contrast learning images 300, 310, and 320 are all projected on one surface, the image conversion device 100 selects a maximum value of brightness values of a plurality of pixels having an overlapping position as a brightness value of the corresponding pixel of the MIP image 330.

The embodiment shows an example of generating the MIP image 330 with respect to the 3 contrast learning images 300, 310, and 320 for convenience of explanation, but the number of contrast learning images 300, 310, and 320 used to generate the MIP image 300 may vary according to an embodiment, such as two or four or more.

As an embodiment, the plurality of contrast learning images 300, 310, and 320 used to generate the MIP image 330 may be a plurality of contrast images captured for a certain period of time after administration of the contrast medium. For example, the image conversion device 100 may select T1 images of MRI captured by administering the contrast medium as the plurality of contrast learning images 300, 310, and 320 used to generate the MIP image 300. As another example, T2 and FIAIR images may be additionally used, but in this case, there is a disadvantage that an image acquisition time is long, which is burdensome to a patient.

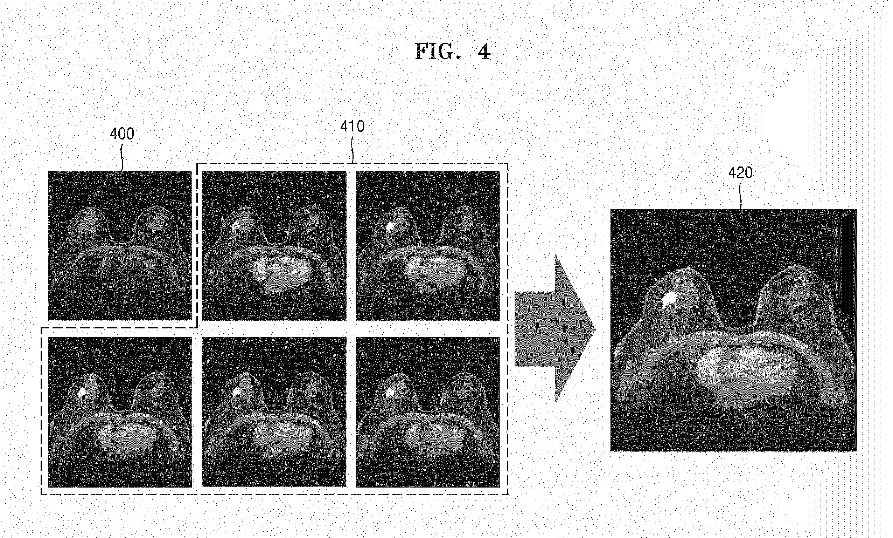

FIG. 4 is a diagram showing another example of a method of generating an MIP image according to an embodiment. Referring to FIG. 4, the image conversion device 100 may generate an MIP image 420 by using a plurality of contrast learning images 410 and a non-contrast learning image 400 together. In other words, when the non-contrast learning image 400 and the plurality of contrast learning images 410 are projected on the same plane, the image conversion device 100 may select a pixel having the greatest brightness value among a plurality of pixels existing at the same location to generate the MIP image 420.

A part (or a pixel) that appears brighter in the non-contrast image 400 than in the contrast image 410 may exist. Therefore, when the deep learning network 200 is trained by generating the MIP image 420 by using the non-contrast learning image 410 together, thereby obtaining a contrast image (i.e., a prediction image) having a clearer contrast.

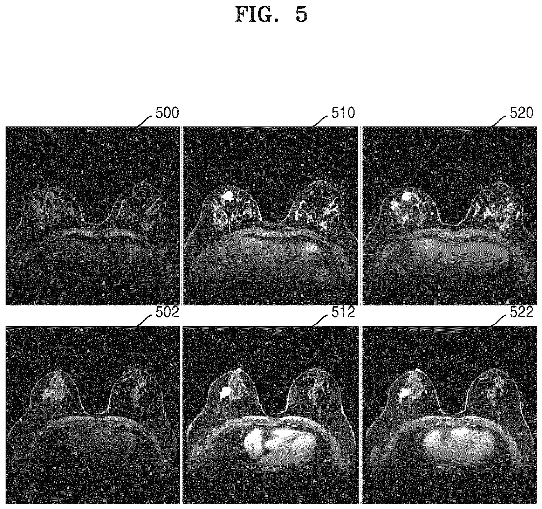

FIG. 5 is a diagram showing a result of a deep learning network trained by using an MIP image according to an embodiment. Referring to FIG. 5, the deep learning network 200 may be trained by using MIP images 510 and 512 as ground truth. The deep learning network 200 trained by using the MIP images 510 and 512 receives and converts non-contrast images 500 and 520 into contrast images 520 and 522, respectively. It may be seen that the contrast images 520 and 522 output by the deep learning network 200 and the MIP images 510 and 512 which are ground truth are almost identical when the contrast images 520 and 522 are compared with the MIP images 510 and 512.

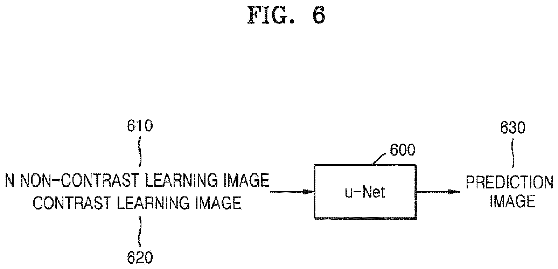

FIG. 6 is a diagram showing an example of implementing a deep learning network in a U-Net according to an embodiment. Referring to FIG. 6, the deep learning network may be implemented as a U-Net 600. The U-Net 600 may be trained with learning data including a dataset of a plurality of non-contrast learning images 610 and one contrast learning image 620. For example, the three sequentially captured non-contrast learning images 610 are input to the U-Net 600, and the U-Net 600 is trained by comparing a prediction image 630 output by the U-Net 600 with a contrast learning image 620 which is ground truth of the learning data such that an error between the prediction image 630 and the contrast learning image 620 is minimized. When three non-contrast images are input to the U-Net 600 of which training is completed, the U-Net 600 outputs the prediction image 630 (i.e., a contrast image). As an embodiment, the contrast learning image 620 of the learning data used for training of the U-Net 600 may be an MIP image described with reference to FIG. 3 or 4.

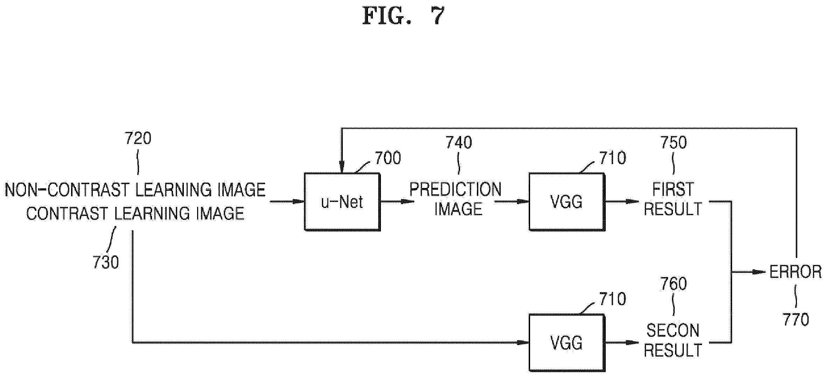

FIG. 7 is a diagram showing an example of a method of training a deep learning network according to an embodiment. Referring to FIG. 7, a deep learning network implemented as a U-Net 700 may be trained by using a visual geometry group (VGG) network 710. In the embodiment, it is assumed that the VGG network 710 has been previously trained with various images. For example, the VGG network 710 may be previously trained to classify images of various objects such as dogs, cats, and cars.

The image conversion device 100 obtains a prediction image 740 by inputting a non-contrast learning image 720 of learning data to the U-Net 700. The image conversion device 100 obtains a first result 750 by inputting the prediction image 740 output by the U-Net 700 to the VGG network 710. In addition, the image conversion device 100 obtains a second result 760 by inputting a contrast learning image 730 which is ground truth of the learning data, to the VGG network 710. The prediction image 740 and the contrast learning image 730 are input to the same network that is the VGG network 710.

The image conversion device 100 determines an error 770 between the first result 750 and the second result 760 obtained by inputting the prediction image 740 and the contrast learning image 730 to the VGG network 710, respectively, and trains the U-Net 700 so that the error 770 is minimized. The image conversion device 100 may obtain a contrast image (i.e., the prediction image 740) by inputting a non-contrast image to the U-Net 700 of which training is completed. As an embodiment, the contrast learning image 730 of the learning data may be an MIP image described with reference to FIG. 3 or 4. As an embodiment, the non-contrast learning images 720 of the learning data may be a plurality of non-contrast images described with reference to FIG. 6.

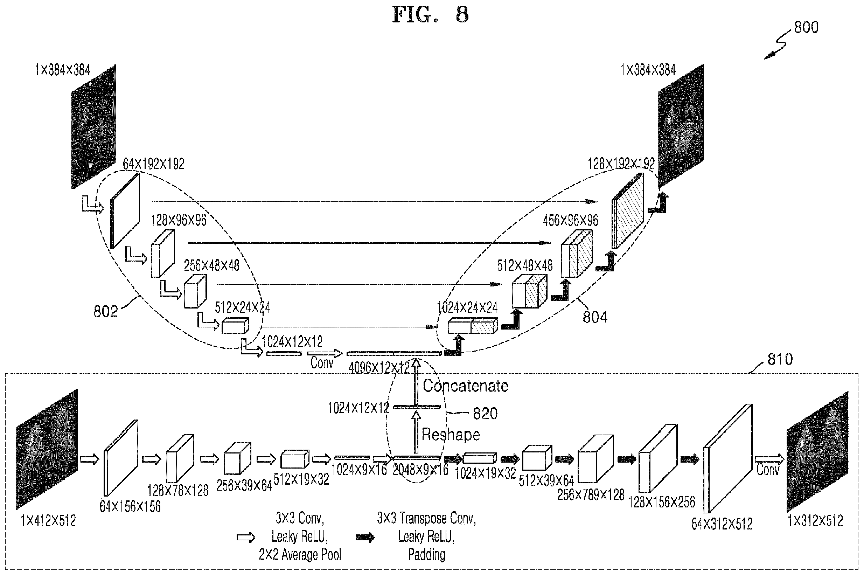

FIG. 8 is a diagram showing another example of a method of training a deep learning network according to an embodiment. Referring to FIG. 8, the deep learning network is implemented as a U-Net 800 including a contracting path 802 and an expanding path 804. An auto encoder 810 may be used for training of the U-Net 800. The auto encoder 810 is trained by restoring an output image as close as possible to an input image when the image is input. The auto encoder 810 itself is already widely known, and thus, an additional description thereof is omitted.

The auto encoder 810 of the embodiment uses a diffusion weighted imaging (DWI) image among MRI images as learning data. When training of the auto encoder 810 is completed, features of a middle layer of a network of the auto encoder 810 are input to the expanding path 804 of the U-Net 800 (820). That is, features of the middlemost part of layers of the auto encoder 810 are extracted and input to the expanding path 804 of the U-Net 800.

A data format of features extracted from the auto encoder 810 may be different from a data format of features received by the expanding path 804 of the U-Net 800, and thus, it is necessary to transform the features extracted from the auto encoder 810 to fit the data format of the U-Net 800. For example, when the features extracted from the middle part of the auto encoder 810 are in the form of a 9*16 array and features of an input end of the expanding path 804 of the U-Net 800 are in the form of a 12*12 array, the size of the features extracted from the auto encoder 810 is converted from 9*16 to 12*12.

The U-Net 800 performs learning by adding the features of the auto encoder 810 to the expanding path 804, thereby learning contrast features included in the DWI image together, and thus, a more accurate contrast image may be generated.

As an embodiment, learning data input to the U-Net 800 may include a dataset of a plurality of non-contrast learning images and one contrast learning image as described with reference to FIG. 6. As an embodiment, a contrast learning image of the learning data of the U-Net 800 may be an MIP image described with reference to FIG. 3 or 4.

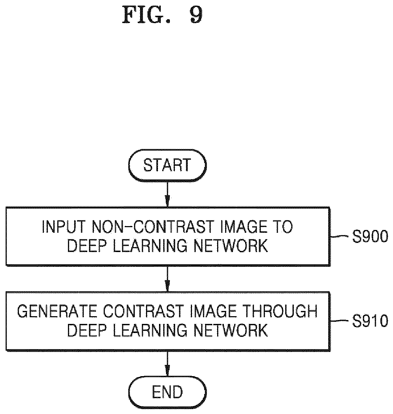

FIG. 9 is a flowchart illustrating an example of a method of converting a non-contrast image into a contrast image according to an embodiment. Referring to FIG. 9, when the non-contrast image is received, the image conversion device 100 inputs the non-contrast image to a deep learning network (S900). The image conversion device 100 obtains the contrast image with respect to the non-contrast image through the deep learning network (S910).

The deep learning network for converting the non-contrast image into the contrast image may be trained in various ways and generated. As an embodiment, the image conversion device 100 may select a plurality of contrast images captured after administrating a contrast medium as a contrast learning image, generate a MIP image including pixels having the greatest brightness value among pixels of the plurality of contrast learning images, and train the deep learning network by using learning data including a non-contrast learning image and an MIP image. Examples in this regard are shown in FIGS. 2 to 5.

As an embodiment, the image conversion device 100 may implement a deep learning network as a U-Net. The image conversion device 100 may train the U-Net by using learning data including a dataset of a plurality of non-contrast learning images and one contrast learning image. An example in this regard is shown in FIG. 6. Alternatively, the image conversion device 100 may obtain a first output value by inputting a contrast prediction image obtained by inputting a non-contrast learning image of the learning data to a deep learning network to a VGG network, obtain a second output value by inputting the contrast learning image to the VGG network, and train the U-Net such that a difference between the first output value and the second output value is minimized. An example in this regard is shown in FIG. 7. Alternatively, the image conversion device 100 may train an auto encoder by using learning data including a DWI image, and train the U-Net by inputting features of a middle layer of a network of the completely trained auto encoder to an expanding path of the U-Net. An example in this regard is shown in FIG. 8.

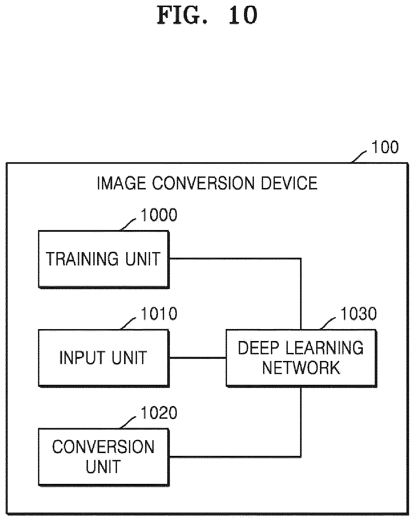

FIG. 10 is a diagram showing the configuration of an example of an image conversion device according to an embodiment. Referring to FIG. 10, the image conversion device 100 includes a training unit 1000, an input unit 1010, a conversion unit 1020, and a deep learning network 1030. As an embodiment, the image conversion device 100 may be implemented as a computing device including a memory, a processor, and an input/output device. In this case, each configuration may be implemented as software and loaded into the memory, and then performed by the processor.

The training unit 1000 trains the deep learning network 1030. The training unit 100 may train the deep learning network 1030 with learning data including one or more contrast learning images and one or more non-contrast learning images. As an embodiment, in order to improve the performance of the deep learning network 1030, the training unit 1000 may use an MIP image described with reference to FIGS. 3 to 5 as the learning data. As an embodiment, the training unit 1000 may train a U-Net by using a plurality of non-contrast images, a VGG network, or an auto encoder after implementing the deep learning network as the U-Net as described with reference to FIGS. 6 to 8.

The input unit 1010 inputs a non-contrast image to the deep learning network 1030. For example, the non-contrast image may be in a data format of digital imaging and communications in medicine (DICOM).

The conversion unit 1020 converts the non-contrast image into a contrast image and outputs the contrast image by using the deep learning network 1030.

The disclosure may also be implemented as computer-readable program codes on a computer-readable recording medium. The computer-readable recording medium includes all types of recording devices in which data readable by a computer system is stored. Examples of the computer-readable recording medium include ROM, RAM, CD-ROM, magnetic tape, floppy disk, an optical data storage device, etc. In addition, the computer-readable recording medium is distributed to computer systems connected over a network, and computer-readable codes may be stored and executed in a distributed manner.

According to an embodiment, a non-contrast image captured without administration of a contrast medium may be converted into a contrast image through a deep learning network. According to an embodiment, the performance of the deep learning network may be improved by using an MIP image generated by processing a plurality of contrast images as learning data instead of using the contrast images as they are as the training data. As an embodiment, the performance of the deep learning network may be improved by implementing the deep learning network as a U-Net and using a VGG network or an auto encoder for training the U-Net.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the spirit and scope of the disclosure as defined by the following claims.

## Claims

1. Image conversion method performed by an image conversion device, the image conversion method comprising:
inputting a non-contrast image to a deep learning network; and
generating and outputting a contrast image through the deep learning network,
wherein the deep learning network is trained with learning data comprising one or more contrast learning images and one or more non-contrast learning images.

2. Image conversion method of claim 1, wherein the non-contrast image is a magnetic resonance imaging (MRI) image.

3. Image conversion method of claim 1 or 2, further comprising:
training the deep learning network,
wherein the training includes:
selecting a plurality of contrast images captured after administration of a contrast medium as a plurality of contrast learning images;
generating a maximum intensity projection (MIP) image including pixels having a greatest brightness value among pixels of the plurality of contrast learning images; and
training the deep learning network by using learning data including the one or more non-contrast learning images and the MIP image.

4. Image conversion method of claim 3, wherein the generating of the MIP image includes selecting a pixel having a greatest brightness value among pixels of the one or more non-contrast learning images and pixels of the plurality of contrast learning images.

5. Image conversion method of claim 3 or 4, wherein the selecting of the plurality of contrast learning images includes selecting T1 images of MRI captured by administrating the contrast medium as the plurality of contrast learning images.

6. Image conversion method of any of claims 1 to 5, wherein the deep learning network is a U-Net.

7. Image conversion method of any of claims 1 to 6, further comprising training the deep learning network, wherein the training includes training the deep learning network by using learning data including a dataset of a plurality of non-contrast learning images and one contrast learning image.

8. Image conversion method of any of claims 1 to 7, further comprising training the deep learning network, wherein the training includes:
obtaining a first output value by inputting a contrast learning image of learning data to a visual geometry group (VGG) network;
obtaining a contrast prediction image by inputting a non-contrast learning image of the learning data to the deep learning network and obtaining a second output value by inputting the contrast prediction image to the VGG network; and
training the deep learning network such that a difference between the first output value and the second output value is minimized.

9. Image conversion method of any of claims 1 to 8, wherein the contrast learning image is an MIP image including pixels having a greatest brightness value among pixels of a plurality of contrast images.

10. Image conversion method of any of claims 6 to 9, further comprising training the deep learning network, wherein the training includes:
training an auto encoder by using learning data including a diffusion weighted imaging (DWI) image; and
training the U-Net by inputting features of a middle layer of a network of the auto encoder that has been trained to an expanding path of the U-Net.

11. Image conversion device comprising:
an input unit (1010) configured to input a non-contrast image to a deep learning network (1030); and
a conversion unit (1020) configured to generate and output a contrast image through the deep learning network (1030),
wherein the deep learning network (1030) is trained with learning data comprising one or more contrast learning images and one or more non-contrast learning images.

12. Image conversion device of claim 11, further comprising a training unit (1000) configured to train the deep learning network.

13. Image conversion device of claim 12, wherein the training unit is configured to generate a maximum intensity projection (MIP) image including pixels having a greatest brightness value among pixels of a plurality of contrast learning images obtained by capturing after administration of a contrast medium, and train the deep learning network by using learning data including the MIP image and a non-contrast learning image.

14. Image conversion device of claim 12 or 13, wherein
the deep learning network is a U-Net, and
the training unit is configured to train the deep learning network such that an error between two result values obtained by inputting a prediction image output by the deep learning network and a ground truth image to a predefined visual geometry group (VGG) network, respectively, is minimized, or train the U-Net by inputting features of a middle layer of an auto encoder that has been trained based on a diffusion weighted imaging (DWI) image to an expanding path of the U-Net.

15. Computer-readable recording medium recording thereon a computer program for performing the method of any of claims 1 to 10.
